# EUROPEAN PATENT SPECIFICATION

(11) **EP 4 475 831 B1**
(45) Date of publication and mention of the grant of the patent: **21.10.2026**
(21) Application number: 23709522.9
(22) Date of filing: 07.02.2023
(51) Int. Cl.: A61K 31/185, A61P 25/28

(54) **ALZ-801 FOR USE IN TREATING A COVID-19 ASSOCIATED NEUROLOGICAL SYMPTOM**
ALZ-801 ZUR VERWENDUNG IN DER BEHANDLUNG EINES COVID-19 ASSOZIIERTEN NEUROLOGISCHEN SYMPTOMS
ALZ-801 DESTINÉ AU TRAITEMENT D'UN SYMPTOME NEUROLOGIQUE ASSOCIÉ AVEC COVID-19

(30) Priority: 07.02.2022 US 202263307585 P; 19.09.2022 US 202263407816 P; 30.09.2022 US 202263412106 P
(43) Date of publication of application: 18.12.2024
(73) Proprietor: Alzheon, Inc., Framingham, MA 01701 (US)
(72) Inventor: ABUSHAKRA, Susan, Framingham, MA 01701 (US); HEY, John, Framingham, MA 01701 (US); TOLAR, Martin, Framingham, MA 01701 (US)
(74) Representative: Potter Clarkson
(86) International application number: PCT/US2023/012501
(87) International publication number: WO 2023/150381

(56) References cited:
- WO-A1-2017/044840
- WO-A1-2020/098492
- JOHN A. HEY ET AL: "Clinical Pharmacokinetics and Safety of ALZ-801, a Novel Prodrug of Tramiprosate in Development for the Treatment of Alzheimer's Disease", CLINICAL PHARMACOKINETICS., vol. 57, no. 3, 23 October 2017 (2017-10-23), NZ, pages 315 - 333, XP055633070, ISSN: 0312-5963, DOI: 10.1007/s40262-017-0608-3
- TOLAR MARTIN ET AL: "Neurotoxic Soluble Amyloid Oligomers Drive Alzheimer's Pathogenesis and Represent a Clinically Validated Target for Slowing Disease Progression", INTERNATIONAL JOURNAL OF MOLECULAR SCIENCES, vol. 22, no. 12, 1 January 2021 (2021-01-01), pages 6355, XP093021547, Retrieved from the Internet <URL:https://www.ncbi.nlm.nih.gov/pmc/articles/PMC8231952/pdf/ijms-22-06355.pdf> DOI: 10.3390/ijms22126355
- XU ET AL: "A SAGE study of apolipoprotein E3/3, E3/4 and E4/4 allele-specific gene expression in hippocampus in Alzheimer disease", MOLECULAR AND CELLULAR NEUROSCIENCES, SAN DIEGO, US, vol. 36, no. 3, 27 October 2007 (2007-10-27), pages 313 - 331, XP022317993, ISSN: 1044-7431, DOI: 10.1016/J.MCN.2007.06.009
- TIAN JIDE ET AL: "GABAA-Receptor Agonists Limit Pneumonitis and Death in Murine Coronavirus-Infected Mice", VIRUSES, vol. 13, no. 6, 23 May 2021 (2021-05-23), pages 966, XP093009129, Retrieved from the Internet <URL:https://www.ncbi.nlm.nih.gov/pmc/articles/PMC8224554/pdf/viruses-13-00966.pdf> DOI: 10.3390/v13060966

## Description

### RELATED APPLICATIONS

This application claims the benefit of priority to U.S. Provisional Application No. 63/307,585, filed February 7, 2022; U.S. Provisional Application No. 63/407,816, filed September 19, 2022; and U.S. Provisional Application No. 63/412,106, filed September 30, 2022.

### BACKGROUND

Infection with the novel coronavirus SARS-CoV-2 which causes severe acute respiratory syndrome, was first identified in late 2019 and called COVID-19. COVID-19 infections reached pandemic status by early 2020, and have since affected approximately 400 million people worldwide, leading to approximately 6 million deaths. In the US alone, symptomatic COVID-19 infections affected an estimated 124 million people, leading to approximately 7.5 million hospitalizations and 1 million deaths.

The underlying mechanisms of CNS dysfunction in COVID-19 have been investigated in brain tissue from autopsy material, and from fluid biomarkers obtained from hospitalized COVID-19 patients. Neuropathological studies of patients who died with COVID-19 have shown evidence of focal amyloid deposits even in young patients (see e.g., Rhodes et al. β-Amyloid Deposits in Young COVID Patients, Preprint online at Lancet 2022), and of inflammasome activation and hyper-phosphorylated tau (see e.g., Reiken et al. Alzheimer's Dement. 2022;1-11).

A large study in hospitalized patients in the US showed increased plasma levels of p-tau, increase p-tau/Abeta42, increased GFAP and t-tau elevations. See e.g., Frontera et al. Alzheimer's Dement. 2021;1-12. Similar finding of increased p-tau, p-tau/Abeta42 and t-tau was reported from Spain. See e.g., Matias-Guiu et al. Front. Psychol. 2021; 12:724022. Increased GFAP, a marker of astrocyte activation, is a stress response to increased amyloid levels; and p-tau is a marker of neuronal injury frequently seen in neurodegenerative diseases, such as Alzheimer's disease. These collective data suggest that the stressors of acute systemic inflammation, activated endothelium and complement, hypoxia, and/or hypoxic-ischemic insults induce an increase in Abeta42 production and tau hyperphosphorylation resulting in increased Abeta42 and p-tau levels in brain and CSF.

Therapeutic treatments that can inhibit the production of Abeta42, or inhibit the formation of toxic Abeta oligomers, reduce p-tau levels, and/or lower the p-tau/Abeta42 ratio, should therefore ameliorate neuronal injury, rescue neuronal function and improve the cognitive dysfunction related to COVID-19 associated neurological symptoms. It is known that ALZ-801 is effective in the treatment of Alzheimer's disease; the role of alleles of Apolipoprotein E (APOE) is also known: WO2017044840; JOHN A. HEY ET AL: "Clinical Pharmacokinetics and Safety of ALZ-801, a Novel Prodrug of Tramiprosate in Development for the Treatment of Alzheimer's Disease", CLINICAL PHARMACOKINETICS, vol. 57, no. 3, 23 October 2017 (2017-10-23), pages 315-333; TOLAR MARTIN ET AL: "Neurotoxic Soluble Amyloid Oligomers Drive Alzheimer's Pathogenesis and Represent a Clinically Validated Target for Slowing Disease Progression", INTERNATIONAL JOURNAL OF MOLECULAR SCIENCES, vol. 22, no. 12, 1 January 2021 (2021-01-01), page 6355; XU ET AL: "A SAGE study of apolipoprotein E3/3, E3/4 and E4/4 allele-specific gene expression in hippocampus in Alzheimer disease", MOLECULAR AND CELLULAR NEUROSCIENCES, SAN DIEGO, US, vol. 36, no. 3, 27 October 2007 (2007-10-27), pages 313-331.

### SUMMARY

The invention is defined in the appended set of claims.

Any references in the description to methods of treatment refer to the compounds, pharmaceutical compositions and medicaments of the present invention for use in a method for treatment of the human (or animal) body by therapy.

It has now been found that ALZ-801, a valine prodrug of tramiprosate, is effective at reducing plasma p-tau₁₈₁ and the p-tau₁₈₁/Abeta42 ratio in subjects with early Alzheimer's disease (AD) having at least one ApoE4 allele. See e.g., **FIG. 1** where an 18%, 29%, and 41% reduction in plasma p-tau₁₈₁ was observed at 13 weeks, 26 weeks, and 52 weeks respectively, from treatment with 265 mg ALZ-801 administered twice daily. A similar trend is shown in **FIG. 2**, where a 21%, 30%, and 31% reduction in plasma p-tau₁₈₁ /Abeta42 was observed at 13 weeks, 26 weeks, and 52 weeks respectively. Reductions in plasma Abeta40 and Abeta42 were also observed at week 52. See e.g., **FIG. 3** and **FIG. 4****.**

Described are methods for reducing the levels of p-tau in a subject by administering a therapeutically effective amount of ALZ-801, in particular p-tau₁₈₁, and further in particular the plasma levels of p-tau₁₈₁.

Also described are methods for reducing the ratio of p-tau/Abeta42 in a subject by administering a therapeutically effective amount of ALZ-801, in particular the ratio of p-tau₁₈₁/Abeta42, and further in particular the ratio of p-tau₁₈₁/Abeta42 in plasma.

Also described
are methods for reducing the levels of Abeta40 in a subject by administering a therapeutically effective amount of ALZ-801, in particular plasma levels of Abeta40.

Also described are methods for reducing the levels of Abeta42 in a subject by administering a therapeutically effective amount of ALZ-801, in particular plasma levels of Abeta42.

Also described are methods of treating conditions associated with elevated p-tau levels (e.g., elevated p-tau₁₈₁ levels and/or elevated plasma p-tau₁₈₁ levels), elevated Abeta40 (e.g., elevated plasma Abeta40 levels), elevated Abeta42 (e.g., elevated plasma Abeta42 levels), and/or p-tau/Abeta42 ratio (e.g., p-tau₁₈₁/Abeta42 ratio and/or plasma p-tau₁₈₁/Abeta42 ratio) by administering a therapeutically effective of ALZ-801. Such conditions include COVID-19 associated neurological symptoms.

### BRIEF DESCRIPTION OF THE FIGURES

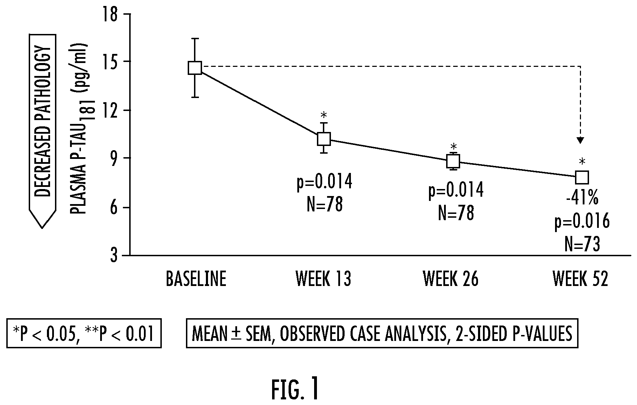

**FIG. 1** illustrates the effect on plasma p-tau₁₈₁ following a 52 week treatment with 265 mg ALZ-801 administered orally twice daily to subjects who are APOE4⁺ and have early AD.

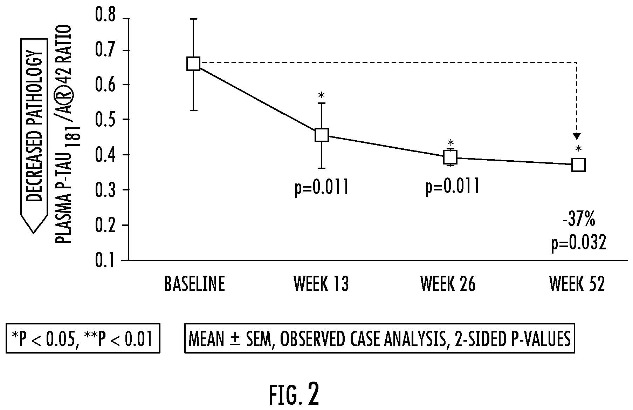

**FIG. 2** illustrates the effect on the p-tau₁₈₁/Abeta42 ratio following a 52 week treatment with 265 mg ALZ-801 administered orally twice daily to subjects who are APOE4⁺ and have early AD.

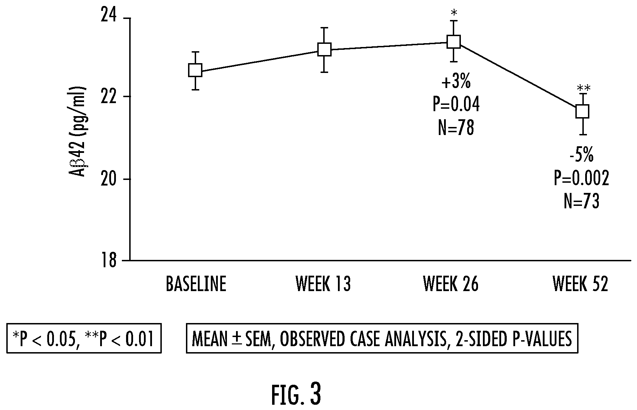

**FIG. 3** illustrates the effect on plasma Abeta42 following a 52 week treatment with 265 mg ALZ-801 administered orally twice daily to subjects who are APOE4⁺ and have early AD.

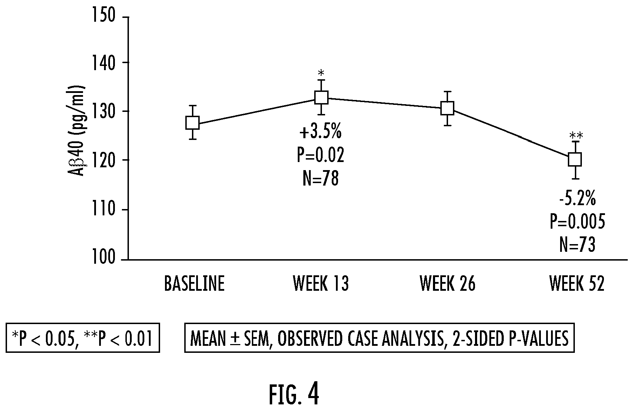

**FIG. 4** illustrates the effect on plasma Abeta40 following a 52 week treatment with 265 mg ALZ-801 administered orally twice daily to subjects who are APOE4⁺ and have early AD.

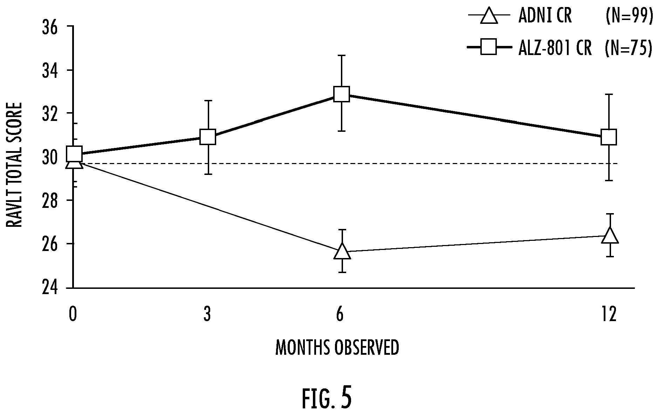

**FIG. 5** illustrates the effect on memory as measured by RAVLT following a 52 week treatment with 265 mg ALZ-801 administered orally twice daily to subjects who are APOE4⁺ and have early AD.

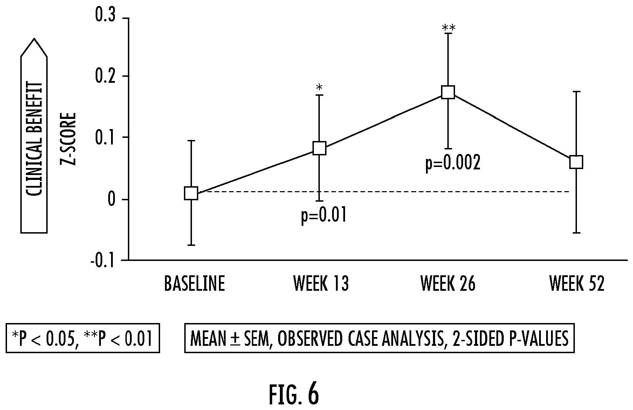

**FIG. 6** illustrates the improvement in cognition following a 52 week treatment with 265 mg ALZ-801 administered orally twice daily to subjects who are APOE4⁺ and have early AD as determined by a composite Z-score based on 3 RAVLT tests and a digit symbol substitution test (DSST).

### DETAILED DESCRIPTION

Described
is a method of reducing the levels (e.g., plasma levels) of p-tau₁₈₁ in a subject comprising administering to the subject a therapeutically effective amount of ALZ-801, or a pharmaceutically acceptable salt thereof.

Described
is a method of reducing the p-tau₁₈₁/Abeta42 ratio (e.g., the plasma p-tau₁₈₁/Abeta42 ratio) in a subject comprising administering to the subject a therapeutically effective amount of ALZ-801, or a pharmaceutically acceptable salt thereof.

Described is a method of reducing the levels (e.g., plasma levels) of Abeta40 in a subject comprising administering to the subject a therapeutically effective amount of ALZ-801, or a pharmaceutically acceptable salt thereof.

Described is a method of reducing the levels (e.g., plasma levels) of Abeta42 in a subject comprising administering to the subject a therapeutically effective amount of ALZ-801, or a pharmaceutically acceptable salt thereof.

ALZ-801 refers to valyl-3-amino-1-propanesulfonic acid (also known as valiltramiprosate), represented by the structure below:

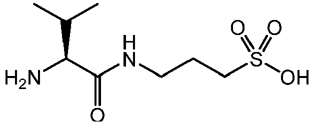

The terms "subject" and "patient" may be used interchangeably, and means a mammal in need of treatment, e.g., companion animals (e.g., dogs, cats, and the like), farm animals (e.g., cows, pigs, horses, sheep, goats and the like) and laboratory animals (e.g., rats, mice, guinea pigs and the like). Typically, the subject is a human in need of treatment.

The term "effective amount" or "therapeutically effective amount" refers to an amount of ALZ-801 or pharmaceutically acceptable salt that will elicit the recited biological or medical response of a subject e.g., a reduction in p-tau₁₈₁, a reduction in Abeta40, a reduction in Abeta42, a reduction in the p-tau₁₈₁/Abeta42, or to treat a recited condition.

As used herein, the term "treat", "treating" or "treatment" means reversing, alleviating, inhibiting, or slowing the progress of a recited condition or one or more symptoms associated therewith.

Described herein is a method of treating a condition associated with elevated p-tau levels (e.g., elevated plasma p-tau levels), elevated Abeta40 levels (e.g., elevated plasma Abeta 40 levels), elevated Abeta42 levels (e.g., elevated plasma Abeta 42 levels), and/or an elevated p-tau/Abeta42 ratio in a subject comprising administering to the subject a therapeutically effective amount of ALZ-801, or a pharmaceutically acceptable salt thereof. In one aspect the method decreases p-tau₁₈₁/Abeta42 ratio. In one aspect the method decreases p-tau₁₈₁/Abeta42 ratio in plasma. In another aspect the method decreases p-tau₁₈₁ levels. In another aspect the method decreases p-tau₁₈₁ plasma levels. In another aspect the method decreases Abeta40 levels. In another aspect the method decreases plasma Abeta40 levels. In another aspect the method decreases Abeta42 levels. In another aspect the method decreases plasma Abeta42 levels.

Conditions associated with elevated p-tau levels, elevated Abeta40 levels, elevated Abeta42 levels, and/or an elevated p-tau/Abeta42 ratio include, but are not limited to, COVID-19 associated neurological symptoms (e.g., encephalopathy such as chronic traumatic encephalopathy, thrombotic strokes, short term memory deficits, cognitive dysfunction such as "Brain Fog", neuropsychiatric disturbances, dizziness, focal symptoms and other neurological symptoms associated with the long-term effects of COVID-19 infection referred to generally as "Long COVID"), traumatic brain injury (acute and/or chronic), single or repetitive brain trauma, mild to severe concussion, hypoxic-ischemic insults post cardiac or cardio-thoracic surgery, post cardiac arrest, and hypertensive vascular dementia. Other conditions include, mild cognitive impairment ("MCI"), vascular dementia, Alzheimer's disease (including early AD, mild to moderate AD, severe AD, and sporadic (non-hereditary) AD), familial (hereditary) age-related cognitive decline, cerebral amyloid angiopathy ("CAA"), hereditary cerebral hemorrhage, senile dementia; Down's syndrome, inclusion body myositis ("IBM"), age-related macular degeneration ("ARMD").

In one aspect, the subject treated herein has AD. Alternatively, the subject treated herein has mild AD. In another alternative, the subject treated herein has mild-to-moderate AD.

In one aspect, the subject treated herein, is of the APOE4/4 or APOE3/4 genotype. Alternatively, the subject treated herein is of the APOE3/4 genotype.

In one aspect, the subject treated herein has memory deficiency attributed to any of the foregoing disclosed conditions and demonstrates an improvement in memory after treatment with ALZ-801. In some aspects the improvement in memory occurs within 26 weeks of treatment. In some aspects the improvement in memory occurs between about 13 and about 26 weeks of treatment.

In one aspect, the subject treated herein, is administered 530 mg ALZ-801/day. Alternatively, the subject treated herein is administered 265 mg ALZ-801 twice daily (BID). In some aspects the ALZ-801 is administered orally. In some aspects the ALZ-801 is administered daily at least until an improvement of the disease or condition is observed. In some aspects the ALZ-801 is continued to be administered daily after an improvement of the disease or condition is observed.

### EXEMPLIFICATION

Evaluation of disease modifying effects from phase 2 trial of ALZ-801 (valiltramiprosate) in subjects with early Alzheimer's disease were conducted. The study enrolled 84 patients, with early AD, who carry the APOE4/4 or APOE3/4 genotype and have an MMSE score ranging from 22 to 30 (CDR-G 0.5 or 1). Each patient received 265 mg ALZ-801 twice daily. The average MMSE score of the subjects was 26 (mean age 69 years, 52% female). Subjects were either amyloid PET positive or A+/T+ on CSF assays. CSF criteria were: Ratio of Aβ42/40 x10 ≤ 0.61, and p-tau₁₈₁ ≥ 61 pg/ml. CSF was evaluated at 52 weeks, and plasma biomarkers at every visit.

All fluid biomarker analyses were performed at the Neurochemistry Laboratory of Dr. Blennow (Molndal, Sweden), and were blinded to subject's demographics or genotype. CSF biomarker assays were analyzed using Lumipulse (Fujirebio) and plasma assays utilized Simoa assays. Cognitive tests include the Rey Auditory Verbal Learning Test (RAVLT: immediate, delayed and recognition memory) and Digit Symbol Substitution Tests (DSST), and a composite Z-score is calculated (3-item RAVLT + DSST). Change from baseline (CBL) analyses were performed on the mITT population and included all observed data, using paired t-tests and 2-sided p-values. The primary biomarker outcome was p-tau₁₈₁.

Of the 84 APOE4 carrier that were enrolled and received ALZ-801, 80 and 75 subjects completed 26 and 52 weeks respectively. In this population, ALZ-801 265 mg BID demonstrated a 41% reduction in plasma p-tau₁₈₁ (p=0.016) at 52 weeks, a 29% reduction in plasma p-tau₁₈₁ (p=0.014) at 26 weeks, and a 18% reduction at 13 weeks (p=0.014). See **FIG 1****.** ALZ-801 also significantly reduced the plasma p-tau₁₈₁/Aβ42 ratio by 37% at 52 weeks (p=0.032), 30% at 26 weeks (p=0.011), and by 21% at 13 weeks (p=0.011). See **FIG 2****.** This is much greater than the annualized ~12% reduction in plasma p-tau₁₈₁ observed in the recent human clinical trials of the anti-amyloid antibody lecanemab (van Dyck, CH et al., N Engl J Med 2023; 388:9-21, DOI: 10.1056/NEJMoa2212948).

Target engagement of ALZ-801 was further validated in this population by data showing an increase in the plasma levels of Abeta40 and Abeta42 at 13-26 weeks, followed by significant 5% reductions in each at 52 weeks (p= 0.002 and p=0.005, respectively). See **FIG**. **3** and **4****.** Mechanistically, ALZ-801 is believed to inhibit β-amyloid oligomer formation in the brain resulting in the inhibition of both formation of oligomers and subsequent aggregation. Aggregation and fibril formation of amyloid-β Abeta40 and Abeta42 peptides are central events in subjects with early Alzheimer's disease. At baseline, untreated subjects possess a certain level of Abeta40 and Abeta42 in both the brain and plasma. When treated with ALZ-801, however, initial increases in plasma Abeta40 and Abeta42 were observed, supporting the belief that amyloid aggregation in the brain is being blocked, and Abeta40 and Abeta42 are being cleared into the plasma. See e.g., the initial surge in plasma Abeta42 and Abeta40 observed in weeks 1-26 and 1-13 of treated subjects as illustrated in **FIG. 3** and **FIG. 4****.** Following this, overall reduction in plasma Abeta40 and Abeta42 was then observed, indicating that ALZ-801 had promoted efficient clearance of soluble amyloid beta species from the brain.

In this study population, ALZ-801 also demonstrated a sustained improvement in memory tests. Clinical outcomes were taken from subjects in the Alzheimer's Disease Neuroimaging Initiative (ADNI). ADNI is a longitudinal database registry of clinical, imaging, and biomarker captured in AD subjects and cognitive normal subjects accepted by the FDA for retrospective research as an external historical control comparator group. Real word evidence (RWE) are data collected on individuals that fall outside of data captured in clinical trials. In this case, we integrated ADNI as a RWE source to serve as an external control arm. As shown by **FIG**. **5****,** treatment with ALZ-801 showed memory improvements up to about 6-months and maintained an improvement over baseline using the Rey's Auditory Verbal Learning Test ("RAVLT"). These results were consistently superior to the cognitive results for AD severity- and genotype-matched, untreated patients as obtained from the ADNI database.

As shown in **FIG**. **6****,** the composite Z-score of cognition of subjects in this clinical trial ((RAVLT memory scores + DSST) showed significant increase at 26 weeks (p=0.002) and remained numerically above baseline after 52 weeks of treatment with ALZ-801.

While certain data presented is specific for p-tau₁₈₁, without being bound by theory, applicants believe other forms of phosphorylated tau protein, e.g., p-tau₂₁₇ and p-tau₂₃₁ will also be reduced by treatment with ALZ-801. Accordingly, the disclosure also provides for the treatment of diseases and conditions associated with abnormally high levels of any of the isoforms of p-tau by the administration of ALZ-801.

In addition, the subjects in this clinical trial, showed an approximate 20% annualized reduction in atrophy of hippocampal volume on ALZ-801 as compared to a well-matched ADNI cohort (p=0.05). This preservation of hippocampal volume showed significant correlations with cognitive benefits (Spearman's 0.27, p = 0.02) suggesting neuroprotection. In contrast, Lecanemab did not demonstrate any significant annualized reduction in hippocampal volume atrophy. Van Dyck, 2022, supra.

Unlike anti-amyloid antibodies, such as Lecanemab, ALZ-801 treatment over 52 weeks also appeared to show an ~15% reduction in ventricular volume expansion as measured by MRI (p=0.08).

While we have described a number of embodiments of this invention, it is apparent that our basic examples may be altered to provide other embodiments that utilize the compounds and methods of this invention. Therefore, it will be appreciated that the scope of this invention is to be defined by the appended claims rather than by the specific embodiments that have been represented by way of example.

Unless otherwise defined, all technical and scientific terms used herein are accorded the meaning commonly known to one with ordinary skill in the art.

## Claims

1. The compound ALZ-801, or a pharmaceutically acceptable salt thereof, for use in treating a COVID-19 associated neurological symptom, wherein the COVID-19 associated neurological symptom is selected from mild cognitive impairment ("MCI"), vascular dementia, Alzheimer's disease including early AD, mild to moderate AD, severe AD, and sporadic (non-hereditary) AD, and familial (hereditary) age-related cognitive decline.

2. The compound for use of claim 1, wherein the treatment reduces p-tau₁₈₁ levels in the subject.

3. The compound for use of claim 1, wherein the treatment reduces p-tau₁₈₁/Abeta42 ratio in the subject.

4. The compound for use of claim 1, wherein the treatment reduces Abeta40 levels in the subject.

5. The compound for use of claim 1, wherein the treatment reduces Abeta42 levels in the subject.

6. The compound for use of any one of Claims 1 to 5, wherein the COVID-19 associated neurological symptom is MCI.

7. The compound for use of any one of Claims 1 to 5, wherein the COVID-19 associated neurological symptom is Alzheimer's disease.

8. The compound for use of Claim 7, wherein the Alzheimer's disease is early Alzheimer's disease or mild to moderate Alzheimer's disease.

9. compound for The use of any one of Claims 1 to 8, wherein the treatment is in a subject having Alzheimer's disease.

10. The compound for use of any one of Claims 1 to 5, wherein the COVID-19 associated neurological symptom is familial (hereditary) age-related cognitive decline.

11. The compound for use of any one of Claims 1 to 10, wherein the p-tau₁₈₁ levels, the Abeta40 levels, the Abeta42 levels, and the p-tau₁₈₁/Abeta42 ratio refer to the levels and ratio in plasma.

12. The compound for use of any one of Claims 1 to 11, wherein the subject is administered 530 mg ALZ-801/day.

13. The compound for use of Claim 12, wherein the subject is administered 265 mg ALZ-801 twice daily.

14. The compound for use of any one of Claims 1 to 13, wherein the subject is of the APOE4/4 or APOE3/4 genotype.

15. The compound for use of Claim 14, wherein the subject is of the APOE3/4 genotype.

## Patentansprüche

1. Verbindung ALZ-801 oder ein pharmazeutisch verträgliches Salz davon zur Verwendung bei einem Behandeln eines mit COVID-19-assoziierten neurologischen Symptoms, wobei das mit COVID-19-assoziierte neurologische Symptom ausgewählt ist aus leichter kognitiver Beeinträchtigung ("MCI"), vaskulärer Demenz, Alzheimer-Krankheit, einschließlich früher Alzheimer-Krankheit, leichter bis mittelschwerer Alzheimer-Krankheit, schwerer Alzheimer-Krankheit und sporadischer (nicht-erblicher) Alzheimer-Krankheit, und familiärem (erblichem) altersbedingtem kognitivem Abbau.

2. Verbindung zur Verwendung nach Anspruch 1, wobei die Behandlung p-tau₁₈₁-Spiegel bei dem Subjekt reduziert

3. Verbindung zur Verwendung nach Anspruch 1, wobei die Behandlung ein p-tau₁₈₁/Abeta42-Verhältnis in dem Subjekt reduziert.

4. Verbindung zur Verwendung nach Anspruch 1, wobei die Behandlung die Abeta40-Spiegel in dem Subjekt reduziert.

5. Verbindung zur Verwendung nach Anspruch 1, wobei die Behandlung die Abeta42-Spiegel in dem Subjekt reduziert.

6. Verbindung zur Verwendung nach einem der Ansprüche 1 bis 5, wobei das mit COVID-19-assoziierte neurologische Symptom MCI ist.

7. Verbindung zur Verwendung nach einem der Ansprüche 1 bis 5, wobei das mit COVID-19-assoziierte neurologische Symptom die Alzheimer-Krankheit ist.

8. Verbindung zur Verwendung nach Anspruch 7, wobei die Alzheimer-Krankheit frühe Alzheimer-Krankheit oder leichte bis mittelschwere Alzheimer-Krankheit ist.

9. Verbindung zur Verwendung nach einem der Ansprüche 1 bis 8, wobei die Behandlung bei einem Subjekt mit Alzheimer-Krankheit erfolgt.

10. Verbindung zur Verwendung nach einem der Ansprüche 1 bis 5, wobei das mit COVID-19 assoziierte neurologische Symptom familiärer (erblicher) altersbedingter kognitiver Abbau ist.

11. Verbindung zur Verwendung nach einem der Ansprüche 1 bis 10, wobei sich die p-tau ₁₈₁-Spiegel, die Abeta40-Spiegel, die Abeta42-Spiegel und das p-tau₁₈₁/Abeta42-Verhältnis auf die Spiegel und das Verhältnis in Plasma beziehen.

12. Verbindung zur Verwendung nach einem der Ansprüche 1 bis 11, wobei dem Subjekt 530 mg ALZ-801/Tag verabreicht werden.

13. Verbindung zur Verwendung nach Anspruch 12, wobei dem Subjekt zweimal täglich 265 mg ALZ-801 verabreicht werden.

14. Verbindung zur Verwendung nach einem der Ansprüche 1 bis 13, wobei das Subjekt den Genotyp APOE4/4 oder APOE3/4 aufweist.

15. Verbindung zur Verwendung nach Anspruch 14, wobei das Subjekt den Genotyp APOE3/4 aufweist.

## Revendications

1. Composé ALZ-801, ou un sel pharmaceutiquement acceptable de celui-ci, pour une utilisation dans le traitement d'un symptôme neurologique associé à la COVID-19, dans lequel le symptôme neurologique associé à la COVID-19 est choisi parmi un trouble cognitif léger (« MCI »), une démence vasculaire, la maladie d'Alzheimer y compris une MA précoce, une MA légère à modérée, une MA sévère et une MA sporadique (non héréditaire), et un déclin cognitif familial (héréditaire) lié à l'âge.

2. Composé pour une utilisation selon la revendication 1, dans lequel le traitement réduit des taux de p-tau₁₈₁ chez le sujet

3. Composé pour une utilisation selon la revendication 1, dans lequel le traitement réduit un rapport p-tau₁₈₁/Abeta42 chez le sujet

4. Composé pour une utilisation selon la revendication 1, dans lequel le traitement réduit des taux d'Abeta40 chez le sujet

5. Composé pour une utilisation selon la revendication 1, dans lequel le traitement réduit des taux d'Abeta42 chez le sujet

6. Composé pour une utilisation selon l'une quelconque des revendications 1 à 5, dans lequel le symptôme neurologique associé à la COVID-19 est un MCI.

7. Composé pour une utilisation selon l'une quelconque des revendications 1 à 5, dans lequel le symptôme neurologique associé à la COVID-19 est la maladie d'Alzheimer.

8. Composé pour une utilisation de la revendication 7, dans lequel la maladie d'Alzheimer est la maladie d'Alzheimer précoce ou la maladie d'Alzheimer légère à modérée.

9. Composé pour une utilisation selon l'une quelconque des revendications 1 to 8, dans lequel le traitement est pour un sujet ayant la maladie d'Alzheimer.

10. Composé pour une utilisation selon l'une quelconque des revendications 1 à 5, dans lequel le symptôme neurologique associé à la COVID-19 est le déclin cognitif familial (héréditaire) lié à l'âge.

11. Composé pour une utilisation selon l'une quelconque des revendications 1 à 10, dans lequel les taux de p-tau₁₈₁, les taux d'Abeta40, les taux d'Abeta42 et le rapport p-tau₁₈₁/Abeta42 se réfèrent aux taux et au rapport dans le plasma.

12. Composé pour une utilisation selon l'une quelconque des revendications 1 à 11, dans lequel 530 mg d'ALZ-801 sont administrés au sujet une fois par jour.

13. Composé pour une utilisation selon la revendication 12, dans lequel 265 mg d'ALZ-801 sont administrés au sujet deux fois par jour.

14. Composé pour une utilisation selon l'une quelconque des revendications 1 à 13, dans lequel le sujet est de génotype APOE4/4 ou APOE3/4.

15. Composé pour une utilisation selon la revendication 14, dans lequel le sujet est de génotype APOE3/4,
